# EUROPEAN PATENT SPECIFICATION

(11) **EP 4 164 338 B1**
(45) Date of publication and mention of the grant of the patent: **28.10.2026**
(21) Application number: 22210279.0
(22) Date of filing: 14.03.2014
(51) Int. Cl.: H05B 45/22, A61B 1/00, H04N 9/64

(54) **ENDOSCOPIC LIGHT SOURCE AND IMAGING SYSTEM**
ENDOSKOPISCHE LICHTQUELLE UND BILDGEBUNGSSYSTEM
SOURCE DE LUMIÈRE ENDOSCOPIQUE ET SYSTÈME D'IMAGERIE

(30) Priority: 15.03.2013 US 201361792165 P
(43) Date of publication of application: 12.04.2023
(62) Document number(s) of the earlier application(s) in accordance with Art. 76 EPC: 14724563.3 / 2 967 299
(73) Proprietor: Stryker Corporation, Portage, MI 49002-9711 (US)
(72) Inventor: RAMESH, Ajay, Pleasanton, California 94588 (US); HU, Perry, San Jose, California 95120 (US); SINGH, Harman, 560093 Bangalore, Kamataka (IN); PANG, Chien Mien, San Jose, California 95128 (US); NAMBAKAM, Vasudev, San Jose, California 95123 (US)
(74) Representative: V.O.

(56) References cited:
- EP-B1- 1 152 642
- US-A1- 2003 030 808
- US-A1- 2003 107 887
- US-A1- 2011 063 427
- US-A1- 2011 208 004

## Description

### BACKGROUND OF THE INVENTION

The present invention relates generally to a solid state system for providing illumination from an external light source through an instrument to an object, such as a patient surgical site. The external light source includes components for providing light in the visible spectrum as well as light in the infrared spectrum.

Endoscopic systems are used to inspect regions within a body during surgery. Endoscopic systems typically include an endoscope, a light source, and an imaging device such as a camera head. Typically, an endoscope includes a rigid or flexible elongated insertion tube equipped with a set of optical fibers that extend from a proximal handle through the endoscope body to a distal viewing tip. An external light source provides light to the optic fibers via a cable that attaches to a post or other structure on the endoscope. The endoscope also receives images and transmits them to the imaging device for providing a image to a monitor or other display apparatus for viewing by a surgeon.

In one commercial embodiment, an endoscopic system includes a solid state light source that generates white light which is conveyed to a distal end of the endoscope via a light guide. The light guide includes multiple fibers and is connected between an output connector of the light source and a light post of the endoscope. The white light illuminates a working area at the distal end of the endoscope. The camera, connected to a handle of the endoscope, generates video signals representative of images at the working area for display on a video monitor.

The light source includes an optical system and a lens array used to collimate light from an LED array. A focusing lens focuses the light onto the light guide. The lenses collect light emitted by LEDs. The lenses may be single lenses, such as single or double aspherics, compound lenses, radiant index type lenses, or combinations of each of these. Other arrangements have lens arrays that are implemented as part of an LED array by adhesion, fusion, or other means. Some arrangements have a rectangular-shaped LED and lens array.

The focal length of the lens and the diameter of the lenses are chosen on the order of a few millimeters. The actual values are selected based on the size of the LED emitting surface which determines the field of view of the lens.

The collected light from the lens array travels to a focusing lens. The focusing lens projects the image of each LED light emitting surface onto an entrance face of the light guide. The image is magnified so that the size is approximately equal to the size of the entrance face of the light guide. The light guide transports the light to the endoscope. The light passes through the endoscope to illuminate a surgical site. Light is reflected off of the surgical site which is received by the endoscope and transmitted to the camera head. The camera head provides images of the surgical site for display on the monitor.

Another endoscopic system that has been designed is described in commonly-owned PCT Application No. WO 2010/059197 A2.

The above-described endoscopic systems do not concern themselves with the ability of excitation of fluorescent markers in an object, such as a body part at a surgical site. While there are systems on the market that do provide excitation light for fluorescent markers, these systems typically use multiple light sources and multiple components to transmit infrared light to the surgical site, and multiple components to separate the fluorescent light emitted by the fluorescent markers. The present disclosure presents a solution to those problems

Further document US 2003/107887 A1 discloses a method of providing color balance in a light source comprising red, green and blue LEDs and a color sensor measuring color temperature and intensity of the emitted light of the LEDs, comparing the measured parameters with parameters for color temperature and intensity pre-set by a control unit, and cause the control unit to adjust the LED currents until the measured parameters correspond to the pre-set parameters.

The present invention as defined in claim 1 is directed to a method of providing color balance in a light source and in claim 6 to a corresponding light source.

Other advantages, objects and/or purposes of the invention will be apparent to persons familiar with constructions of this general type upon reading the following specification and inspecting the accompanying drawings.

### BRIEF DESCRIPTION OF THE DRAWINGS

Fig. 1 is an illustration of an endoscopic camera arrangement which is an embodiment of the present invention.
Fig. 1A is a diagram of a portion of the endoscopic camera arrangement of Fig. 1 and an object with fluorescent markers in it.
Fig. 2 is a block diagram of a light source of the endoscopic system of Fig. 1.
Fig. 3 is a diagrammatic view of an LED and laser diode portion of the light source of Fig. 2.
Fig. 4a is a diagrammatic view of a collimating lens receiving light of a first set of light wavelengths.
Fig. 4b is a diagrammatic view of a collimating lens receiving light of a second set of light wavelengths.
Fig. 4c is a graph showing an excitation light wavelength band and a marker emission light wavelength band.
Fig. 5 is a diagrammatic view of an embodiment of a camera head of the present invention, which includes a trichroic prism for separation of light by wavelength, and image sensors for transformation of light to image signals.
Fig. 6 is a flow chart of a color calibration routine.
Fig. 7 is a flow chart of a color balance routine.
Fig. 8 is a flow chart of an infrared fluorescence routine.

### DETAILED DESCRIPTION

Certain terminology will be used in the following description for convenience and reference only, and will not be limiting. For example, the words "upwardly," "downwardly," "rightwardly," and "leftwardly" will refer to directions in the drawings to which reference is made. The words "inwardly" and "outwardly" will refer to directions toward and away from, respectively, the geometric center of the arrangement, and designated parts thereof. This terminology includes the words specifically mentioned, derivatives thereof, and words of similar import.

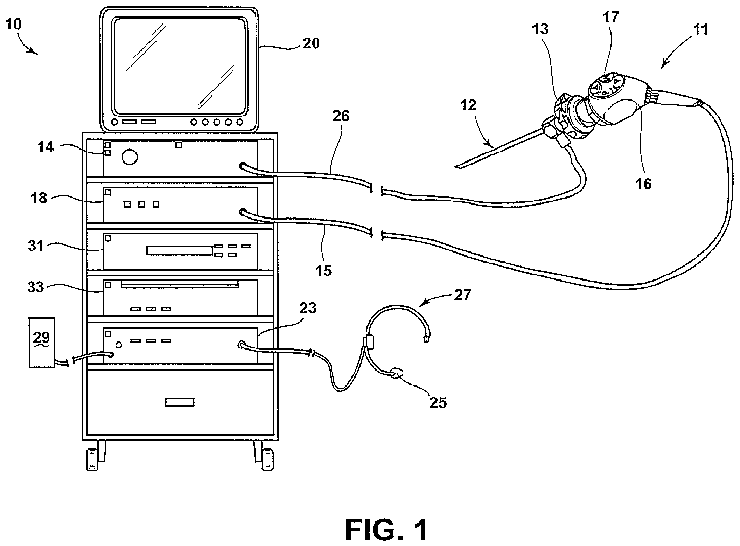

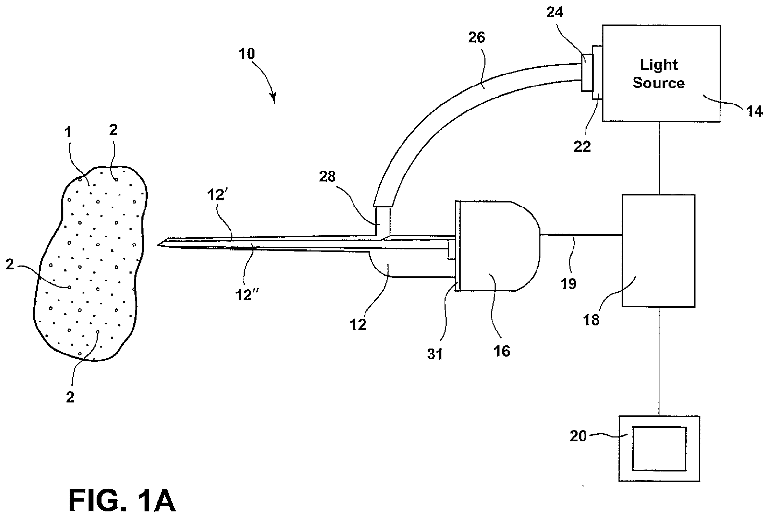

Fig. 1 shows an endoscopic camera arrangement 10, including a scope assembly 11 which may be utilized in endoscopic procedures. The scope assembly 11 incorporates an endoscope or scope 12 which is coupled to a camera head 16 by a coupler 13 located at the distal end of the camera head 16. Light is provided to the scope by a light source 14 via a light guide 26, such as a fiber optic cable. The camera head 16 is coupled to a camera control unit (CCU) 18 by an electrical cable 15. The CCU 18 is connected to, and communicates with, the light source 14. Operation of the camera 16 is controlled, in part, by the CCU 18. The cable 15 conveys video image data from the camera head 16 to the CCU 18 and conveys various control signals bi-directionally between the camera head 16 and the CCU 18, wherein the image data output by the camera head 16 is digital.

A control or switch arrangement 17 is provided on the camera head 16 and allows a user to manually control various functions of the arrangement 10. Voice commands are input into a microphone 25 mounted on a headset 27 worn by the surgeon and coupled to the voice-control unit 23. A hand-held control device 29, such as a tablet with a touch screen user interface or a PDA, may be coupled to the voice control unit 23 as a further control interface. A recorder 31 and a printer 33 are also coupled to the CCU 18. Additional devices, such as an image capture and archiving device, may be included in the arrangement 10 and coupled to the CCU 18. Video image data acquired by the camera head 16 and processed by the CCU 18 is converted to images, which can be displayed on a monitor 20, recorded by recorder 31, and/or used to generate static images, hard copies of which can be produced by the printer 33.

Fig. 1A shows an endoscopic system 10 used to illuminate and receive light from an object 1, such as a surgical site of a patient. The object 1 includes fluorescent markers 2 therein. The fluorescent markers 2 are preferably comprised of indocyanine green (ICG) which is an FDA-approved fluorescent dye for bile duct identification and sentinel lymph node (SLN) identification.

The light source 14 generates white light (a combination of red, green, and blue light) in a first mode, and infrared, blue, and green light in a second mode. In both modes, the light is transmitted to and through an optic lens system 22 which focuses light onto a light pipe 24. The light pipe 24 is preferably five millimeters in diameter and creates a homogeneous light, which is then transmitted to the fiber optic light guide 26. The light guide 26 includes multiple optic fibers and is connected to a light post 28, which is part of the endoscope 12. The endoscope 12 has an illumination pathway 12' and an optical channel pathway 12".

The endoscope 12 includes a notch filter 31, which allows at least 80% of infrared light in a wavelength range of 830 nm to 870 nm to pass therethrough and allows at least 80% of visible light in the wavelength range of 400 nm to 700 nm to pass therethrough, but blocks light having a wavelength of 808 nm. The notch filter 31 should have an optical density of OD5 or higher. Alternatively, the notch filter 31 can be located in the coupler 13.

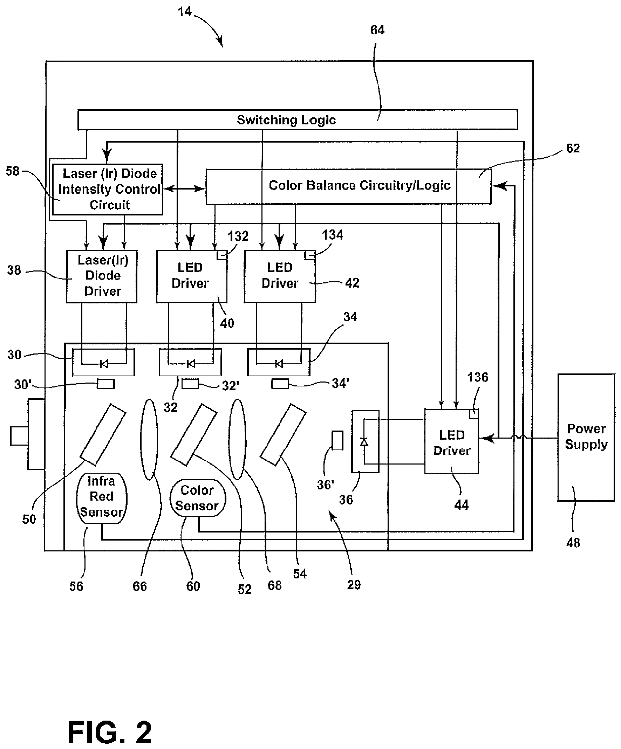

The basic components of the light source 14 are shown in Fig. 2. The light source 14 includes an LED and laser diode section 29, which has a laser diode 30, a first LED 32, a second LED 34, and a third LED 36. The laser diode 30 is preferably an infrared diode (denoted by the letters IR) which emits light having a wavelength in the range of about 805 nm to about 810 nm, and more preferably having a wavelength of about 808 nm. Preferably, the first LED 32 emits light in the blue wavelength spectrum, the second LED 34 emits light in the green wavelength spectrum, and the third LED 36 emits light in the red wavelength spectrum. The infrared laser diode 30 is activated by a laser diode driver 38, the first LED 32 is activated by a first LED driver 40, the second LED 34 is activated by a second LED driver 42, and the third LED 36 is activated by a third LED driver 44. The drivers 38, 40, 42, 44 are each powered by an external power supply 48.

Adjacent the laser diode 30 is a first optical component 30', used to spread the laser light. Adjacent the first LED 32 is a second optical component 32', adjacent the second LED 34 is a third optical component 34', and adjacent the third LED 36 is a fourth optical component 36'. The optical components 32', 34', 36' are for the purpose of decreasing the angles of the paths of the light emitted from the LEDs 32, 34, 36, respectively. The optical components 30', 32', 34', 36' may be any component that is capable of achieving the desired purpose, but preferably are lenses or light pipes.

Adjacent the first optical component 30' is a first dichroic filter 50, adjacent the second optical component 32' is a second dichroic filter 52, and adjacent both the third optical component 34' and the fourth optical component 36' is a third dichroic filter 54. The dichroic filters 50, 52, 54 are each designed to reflect certain light and allow passage of other light therethrough, as described in more detail below.

An infrared sensor 56 is positioned adjacent the first dichroic filter 50, at a location opposite the laser diode 30. The infrared sensor 56 detects the presence of infrared light, and when the presence of infrared light is detected, it provides a signal to a laser diode intensity control circuit 58. The laser diode intensity control circuit 58 is connected to the laser diode driver 38 and controls the intensity of the light emitted from the laser diode 30.

A color sensor 60 is positioned adjacent the second dichroic filter 52, at a location opposite the first LED 32. The color sensor 60 detects light in the visible light wavelength spectrum, and when visible light is detected, it provides a signal to a color balance circuit/logic device 62. The amount of visible light detected is used by the color balance circuit/logic device 62 to provide signals to the LED drivers 40, 42, 44 to adjust the intensity of one or more of the LEDs 32, 34, 36, such that the preferred balance of light in the visible spectrum is achieved.

A switching logic device 64 is provided which switches the light source 14 between the two modes. The first mode is a visible white light mode in which the laser diode 30 is off, the first LED 32 is on, the second LED 34 is on, and the third LED 36 is on. The second mode is an infrared excitation light and background light mode in which the laser diode 30 is on, the first LED 32 is on, the second LED 34 is on, and the third LED 36 is off. The second mode provides infrared light to the surgical site for excitation of fluorescent markers, and provides light in the blue and green visible wavelength spectrums for background light.

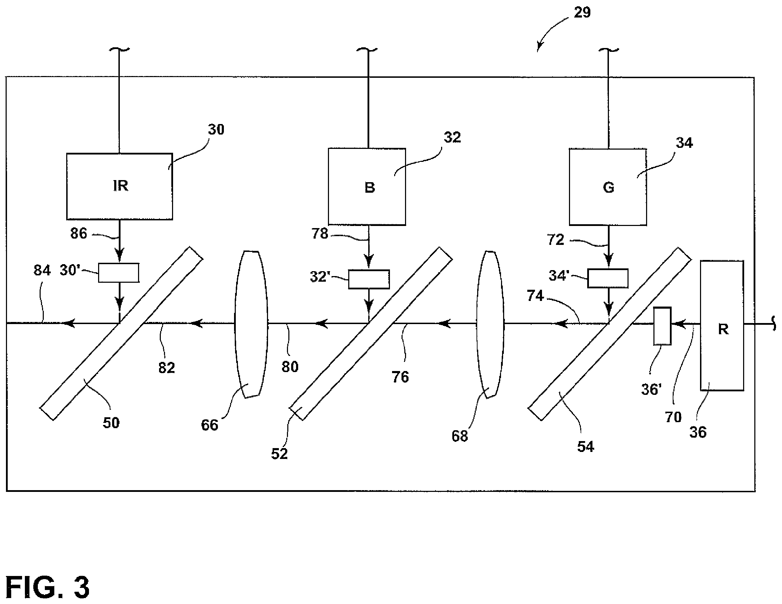

Fig. 3 shows a more detailed view of the LED and laser diode section 29. In this arrangement, the first dichroic filter 50 allows all visible light (i.e. light in the blue, green, and red wavelength spectra) to pass, while reflecting 808-nm infrared light. The second dichroic filter 52 allows light in the red and green wavelength spectra to pass while reflecting light in the blue wavelength spectrum. The third dichroic filter 54 allows light in the red wavelength spectrum to pass, while reflecting light in the green wavelength spectrum. A first optical lens 66 is located between the first dichroic filter 50 and the second dichroic filter 52, and is for focusing light received from the second dichroic filter 52 to be passed to the first dichroic filter 50. A second optical lens 68 is located between the second dichroic filter 52 and the third dichroic filter 54, and is for focusing light received from the third dichroic filter 54 to be passed to the second dichroic filter 52.

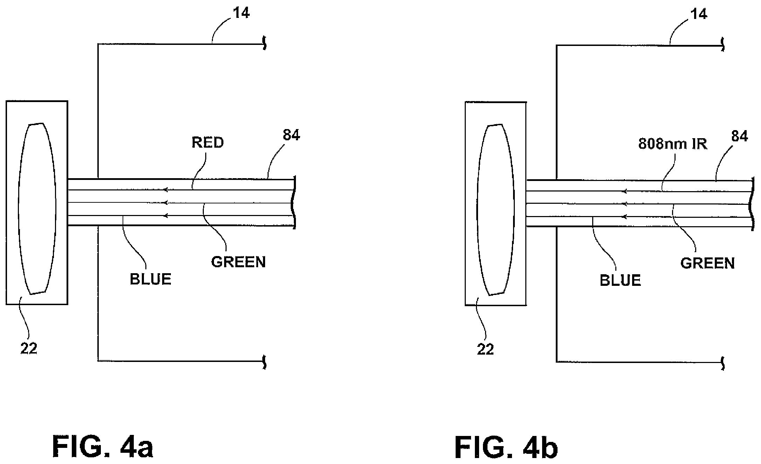

In operation in the first mode, power is not supplied to the laser diode driver 38, but is supplied to the first LED driver 40, the second LED driver 42, and the third LED driver 44. Thus, in this mode, no light is provided by the laser diode 30, but light is provided by the first LED 32, the second LED 34, and the third LED 36. Light in the red wavelength spectrum is emitted from the third LED 36 in the direction of the pathway 70 toward the fourth optical component 36' and the third dichroic filter 54, as shown in Fig. 3. Light in the green wavelength spectrum is emitted from the second LED 34 in the direction of the pathway 72 toward the third optical component 34' and the third dichroic filter 54. Because the third dichroic filter 54 allows red light to pass and reflects green light, the light along the pathway 74 is a mixture of light in the red and green wavelength spectra. This mixture of light from the pathway 74 is focused by the second optical lens 68 and transmitted along the pathway 76 to the second dichroic filter 52. Light in the blue wavelength spectrum is emitted by the first LED 32 along the pathway 78 toward the second optical component 32' and the second dichroic filter 52. Because the second dichroic filter 52 allows red and green light to pass and reflects blue light, the light along the pathway 80 is a mixture of blue, green, and red light. This light is transmitted along the pathway 80 and through optical lens 66, which focuses the light. The focused blue, green, and red light mixture is transmitted along the pathway 82 toward the first dichroic filter 50, which allows blue light, green light, red light to pass. Thus, all of the light transmitted along the pathway 82 passes through the first dichroic filter to an exit pathway 84. The mixture of blue light, green light, and red light, i.e. white light, is transmitted along the exit pathway 84 to the lens system 22, as shown in Fig. 4a.

In the second mode, power is provided to the laser diode driver 38, to the first LED driver 40, and to the second LED driver 42, but is not provided to the third LED driver 44. Thus, the light source 14 provides infrared light (having a wavelength between 710 nm and 820 nm, but preferably having a wavelength of 808 nm), light in the blue wavelength spectrum, and light in the green wavelength spectrum. Because the third LED 36 provides no light in the second mode, there is no light transmitted along pathway 70. The second LED 34 emits light in the green wavelength spectrum along pathway 72 in the direction of the third optical component 34' and the third dichroic filter 54, which reflects the green light. As a result, the green light is transmitted along the pathway 74, to and through the optical lens 68, and along the pathway 76 to the second dichroic filter 52. Light in the blue wavelength spectrum is emitted by the first LED 32 along the pathway 78 in the direction of the second optical component 32' and the second dichroic filter 52. Because the second dichroic filter 52 passes light in the green wavelength spectrum and reflects light in the blue wavelength spectrum, a mixture of blue light and green light is transmitted along the pathway 80, to and through the optical lens 66, and along the pathway 82 to the first dichroic filter 50. The laser diode 30 emits 808-nm infrared light along a pathway 86 in the direction of the first optical component 30' and the first dichroic filter 50. Because the first dichroic filter passes light in the visible wavelength spectrum, and reflects 808-nm infrared light, the result of light transmitted along the exit pathway 84 is a mixture of 808-nm infrared light, green light, and blue light, as shown in Fig. 4b. This mixture of light is transmitted to the lens system 22.

After the light, either in the first mode or in the second mode, passes through the lens system 22, it is transmitted through the light pipe 24, through the fiber optic light guide 26, and to the endoscope 12 via the light post 28. The light transmits through the illumination pathway 12' of the endoscope to the object 1.

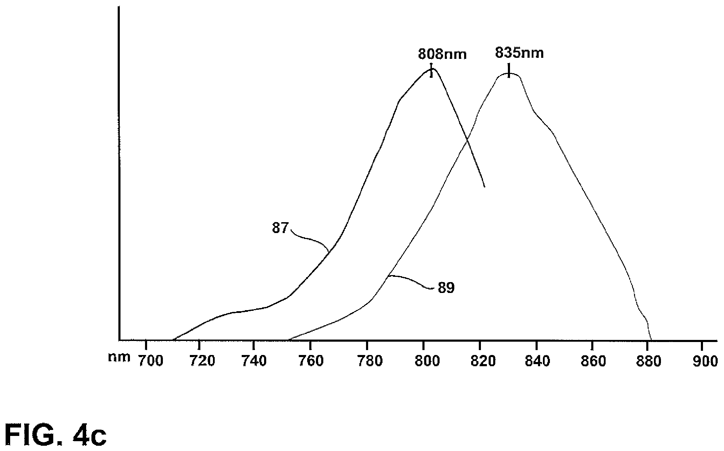

In the first mode, visible light is reflected off of the object 1, a portion of which is received by the endoscope 12, and which is transmitted to the camera head 16 via the optical channel pathway 12". In the second mode, 808-nm infrared light, as well as light in the blue and green wavelength spectra, are transmitted to the object 1. The light in the blue and green wavelength spectra is reflected by the object 1, a portion of which is received by the endoscope 12. In addition, the 808-nm infrared light excites the fluorescent markers 2 in the object. The excitation of the fluorescent markers 2 causes the markers 2 to emit their own light, which is also in the infrared spectrum. The fluorescent marker light preferably has a wavelength between about 760 nm and about 880 nm, with the fluorescent marker light having a peak emission wavelength in the range of about 830 nm to about 840 nm, and more preferably about 835 nm. A graph of the excitation light wavelength band 87 and the fluorescent marker light wavelength band 89 is depicted in Fig. 4c. The excitation infrared light can be distinguished and/or separated from the fluorescent marker light, as discussed below. The emitted marker fluorescent light, and typically a portion of reflected 808-nm excitation light, is received by the endoscope 12. The blue light, green light, and infrared lights are transmitted to the notch filter 31, where 808-nm light is blocked. The unblocked light passes through the notch filter 31 and is transmitted to the camera head 16.

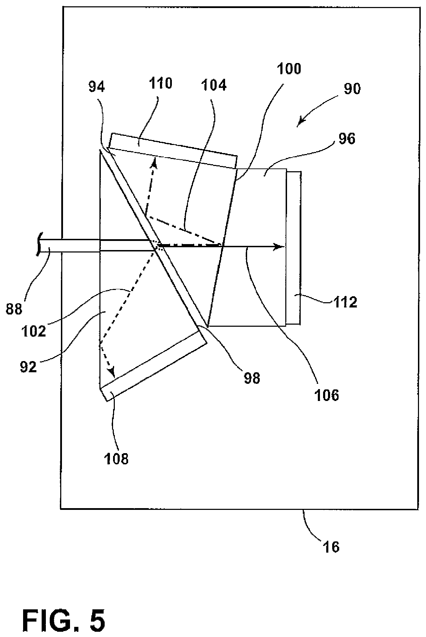

The light, either in the first mode or in the second mode, returns along a path, depicted as part number 88 in Fig. 5, to the camera head 16. The camera head 16 includes, among other components not shown, a trichroic prism 90. The trichroic prism 90 includes a first glass prism 92, a second glass prism 94, and a third glass prism 96. Between the first glass prism 92 and the second glass prism 94 is a first prism filter 98, which may be in the form of a coating on the exterior of the first glass prism 92. The first prism filter 98 reflects blue light, but transmits other light, such as red light, green light, and infrared light. Between the second glass prism 94 and the third glass prism 96 is a second prism filter 100, which may be in the form of a coating on the second glass prism 94. The second prism filter 100 reflects red light and infrared light, but allows the transmission of light in other wavelengths, such as green light. Thus, blue light is transmitted along the pathway 102 as depicted in Fig. 5, red and infrared light are transmitted along the pathway 104, and green light is transmitted along the pathway 106.

A first color sensor 108 is adjacent, and preferably fixedly attached to, the first glass prism 92. A second color sensor 110 is adjacent, and preferably fixedly attached to, the second glass prism 94. A third color sensor 112 is adjacent, and preferably fixedly attached to, the third glass prism 96. The first color sensor 108 is capable of detecting light in the blue wavelength spectrum, the second color sensor 110 is capable of detecting light in the red and infrared wavelength spectra, and the third sensor 112 is capable of detecting light in the green wavelength spectrum. The color sensors 108, 110, 112 receive light and convert the light into electronic signals, which in turn are transmitted to the processor 18 for processing into analog or digital signals of images to be provided for display, such as to monitor 20.

In the first mode, visible light (red, green, blue) is sensed by the color sensors 108, 110, 112, and the resulting visible light signals are sent to the CCU 18, which in turn provides an image of the object 1 to the monitor 20 in full color. In the second mode, the sensors 108, 110, 112 provide signals to the CCU 18 representing a band of infrared light, green light, and blue light. The green light and blue light signals received by the CCU 18 are converted into a black and white image of the object 1 to create a background image. The infrared light signal is processed and sent to a visible "pseudo" color output, in this case the green output of the CCU 18 and to the monitor 20. Thus, in the second mode, the object 1 is presented as a black and white image, while the excited fluorescent markers 2 are displayed as green on the monitor 20.

The switching from the first (white light) mode to the second (IR) mode in both the light source 14 and the CCU 18 can be achieved by use of camera head buttons, a CCU processor 18 touch screen, a light source 14 touch screen, a wireless controller touch screen, a voice control or a foot pedal.

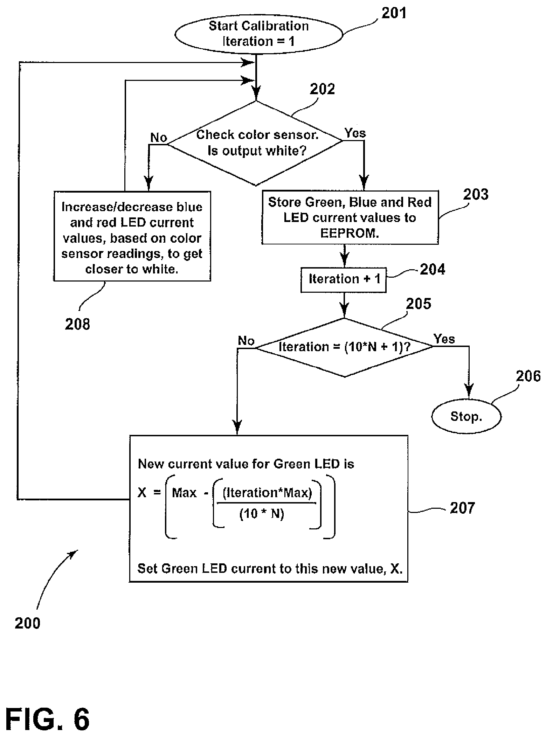

The color balance circuitry/logic device 62 uses an algorithm routine 200 to calibrate the light output. The light source 14 is calibrated once in its life span. As shown in Fig. 6, calibration is started with a first iteration (201). The algorithm utilizes the color sensor 60 to measure the red, green, and blue light output from the LEDs 36, 34, 32, respectively, to determine whether the combination of red, green, and blue light creates white light. If white light is achieved, the LED current values of the red, green, and blue light are stored in a memory device such as an EEPROM, for the given intensity level (202).

In this system, there are 10 brightness levels available to the user, and the number of steps between the present brightness level and the next brightness level is designated as N. Accordingly, after storage of a white light current value, the iteration number is increased by 1 (204), and then the system determines if the iteration number equals 10*N+1 (205). If the iteration number equals 10*N+1, the routine is stopped (206). If not, the new current value for the green LED is set to X (207), with X being: $X = Max - \left(\frac{\left(Iteration*Max\right)}{\left(10*N\right)}\right)$ The system then repeats the above process until the iteration number equals 10*N+1.

If the color sensor 60 does not detect white light at the start of the routine, the red and blue LED current values are increased or decreased, based on readings from the color sensor 60, to adjust the light to be closer to white light (208). The system then starts this portion of routine again to determine if the output is white light (202). The electrical currents supplied to the LEDs 32, 34, 36, are adjusted using a Digital-to-Analog Converter (DAC) 132 for the blue LED 32, a DAC 134 for the green LED 34, and a DAC 136 for the red LED 36.

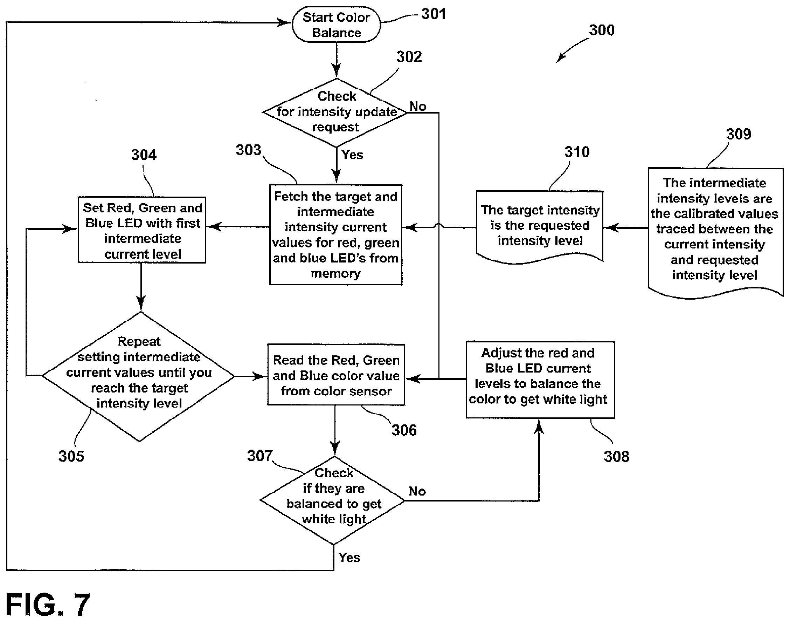

An algorithm routine for color balance is depicted diagrammatically in Fig. 7 and is designated generally as 300. The color balance feature is actively running whenever the unit is on. The color balance routine 300 achieves change in white light intensity without a significant, user-noticeable change in color of the light being output. The algorithm works such that when the color balance is started (301), it is determined whether the user has chosen a white light intensity level (302). If the user has chosen to change the intensity, the target intensity becomes the new, requested intensity (310). The system reads, from the EEPROM, intermediate (i.e., between the present intensity level and the target intensity level) intensity DAC current values for each LED 32, 34, 36, set at the time of calibration (309) to achieve white light for the new intermediate intensity level (303). Then the algorithm adjusts the DAC current values for all the LEDs in adjustments small enough so that the user cannot perceive the small deviations from white light in the color of the light being output (304). These adjustments are repeated until the DAC current values for each LED reach the calibrated DAC current values required for white light for the target intensity level (305).

At all times when the light source 14 is on and the algorithm 300 is not going through the change in light intensity portion of the routine (303, 304, 305), the system is color balancing. To do this, red light, green light, and blue light color values are determined by the color sensor 60 and forwarded to the color balance circuitry/logic device 62 (306). The device 62 determines whether the red, green, and blue lights are balanced to achieve white light (307). If the lights are not balanced to result in white light, the red and blue DAC current levels are adjusted relative to the green DAC current level. This process is then repeated until white light is achieved (308).

The light source 14 being able to communicate with the CCU 18 allows for the light source 14 to automatically change intensity of the white light, turn itself on or off, or change between the first (white light) mode and the second (IR) mode based on the state of the CCU 18. This is particularly useful when a user decides to change the specialty for which the endoscopic camera system 10 is being used. When the user changes the specialty on the CCU 18, the CCU 18 will automatically result in the light source 14 adjusting its brightness for that specialty.

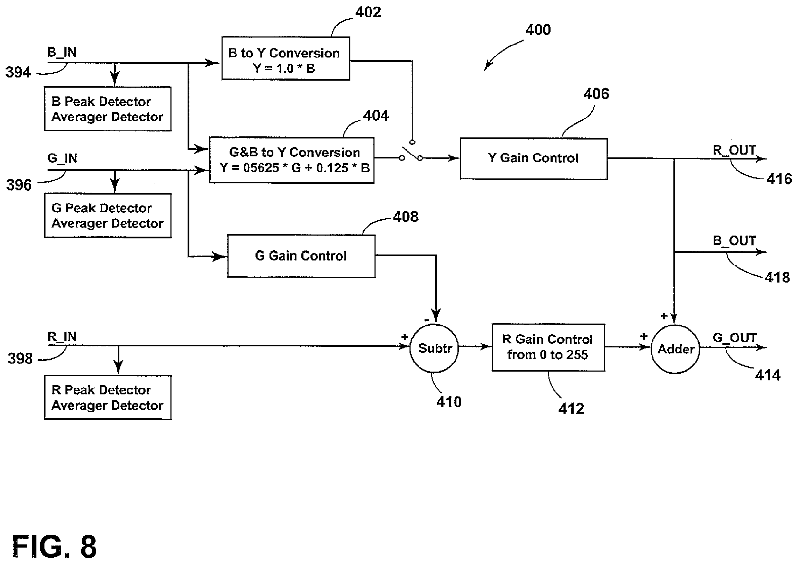

Fig. 8 depicts an infrared fluorescence flow chart. When the light source 14, which is connected to the CCU 18, is switched to the second (IR) mode, an IRF algorithm routine 400 in the CCU 18 is activated. The IRF algorithm causes the following steps to occur. The system has a blue input channel 394, a green input channel 396, and a red input channel 398. Using the blue signal (B) from the blue input channel 394 and the green signal (G) from the green input channel 396, the luminance Y of B is calculated by using the formula Y=1.0*B (402), and/or the luminance Y of G and B combined is calculated by using the formula Y=0.5625*G + 0.125*B (404). The luminance Y of B is calculated in the first (white light) mode and the luminance Y based on B and G is calculated in the second (IR) mode. A suitable luminance gain is then applied to the calculated luminance Y (406) and green input G (408) to enhance the input image.

The trichroic prism 90 is designed to have some overlapping of light spectra to ensure that the trichroic prism 90 does not favor one wavelength over another. Such overlapping, however, can cause some backlight to leak into the red sensor in the second, fluorescent mode. This leakage manifests itself by light in the green spectra leaking into the red sensor. Such leakage errors result in a smearing or portions of the resultant image which are "false." To account for this leakage, the current system includes an algorithm in the CCU to remove some amount of the green light green input value G from a red input value (R). The amount of green light removed from the red light is based on the value of the green input G (410).

A suitable red gain is also applied to the red input channel to enhance the image (412). In the second (IR) mode, the adjusted red channel value is then set to a green output channel 414 and the processed luminance Y is set to red and blue output channels 416, 418.

The described system allows for the traditional use of transmitted light to an object, such as a surgical site, through an endoscope with the reflected light being received and processed. In addition, the described system provides the advantage of providing infrared excitation light to the object for excitation of fluorescent markers. The endoscope is also capable of receiving both the reflected visible light and the excitation light from the object, which is in turn processed and provided as a display in an operating room. The described system uses no additional sensors or processors with respect to the previous white light only system.

Although particular preferred embodiments of the invention have been disclosed in detail for illustrative purposes, it will be recognized that variations or modifications of the disclosed apparatus, including the rearrangement of parts, lie within the scope of the present invention.

## Claims

1. A method of providing color balance in a light source, the method comprising:
providing a first LED, a second LED, and a third LED, the first LED in communication with a first DAC, the second LED in communication with a second DAC, and the third LED in communication with a third DAC;
providing a computing device which is in communication with the first DAC, the second DAC, and the third DAC, the computing device having a memory device having stored therein target intensity values for the first LED, the second LED, and the third LED;
providing a color sensor in communication with the computing device and capable of sensing light in the visible light spectrum and being capable of providing information to the computing device based on sensed light;
the computing device retrieving color values from the color sensor;
the computing device determining whether the color intensity values are balanced to achieve white light;
adjusting the current to the second DAC and third DAC until the color intensity levels are balanced to achieve white light at a white light intensity level;
setting a new red LED current value, a new green LED current value, and a new blue LED current value by reading, from the memory device, an intermediate intensity DAC current value for each LED between its present intensity level and its target intensity level to adjust the white light intensity level toward a target intensity level, wherein the intermediate intensity DAC current values were at the time of calibration stored in the memory device and set to achieve white light for the new intermediate intensity level; and
repeating the setting step until the target intensity level is achieved.

2. The method of claim 1, wherein the target intensity level is chosen by a user.

3. The method of claim 1 or 2, wherein red, green and blue LED current values are stored in the memory device for a plurality of intensity levels.

4. The method of any of claims 1-3, further comprising switching the light source between two modes, wherein a first mode is the visible white light mode in which a laser diode 30 is off, the first LED is on, the second LED is on, and the third LED 36 is on, and wherein a second mode is an infrared excitation light and background light mode in which the laser diode is on, the first LED is on, the second LED is on, and the third LED is off.

5. The method of any of claims 1-4, wherein the first LED is a green led, the second LED is a blue LED, and the third LED is a red LED.

6. A light source comprising:
a first LED (34), a second LED (32), and a third LED (36), the first LED in communication with a first DAC (134), the second LED in communication with a second DAC (132), and the third LED in communication with a third DAC (136) ;
a computing device (62) which is in communication with the first DAC, the second DAC, and the third DAC, the computing device having a memory device having stored therein target intensity values for the first LED, the second LED, and the third LED;
a color sensor (60) in communication with the computing device and capable of sensing light in the visible light spectrum and being capable of providing information to the computing device based on sensed light;
the computing device being configured to:
retrieve color values from the color sensor;
determine whether the color intensity values are balanced to achieve white light;
adjust the current to the second DAC and third DAC until the color intensity levels are balanced to achieve white light at a white light intensity level;
set a new red LED current value, a new green LED current value, and a new blue LED current value by reading, from the memory device, an intermediate intensity DAC current value for each LED between its present intensity level and its target intensity level to adjust the white light intensity level toward a target intensity level, wherein the intermediate intensity DAC current values were at the time of calibration stored in the memory device and set to achieve white light for the new intermediate intensity level; and
repeat the setting step until the target intensity level is achieved.

7. The light source of claim 6, wherein the target intensity level is user selectable.

8. The light source of claim 6 or 7, wherein red, green and blue LED current values are stored in the memory device for a plurality of intensity levels.

9. The light source of any of claims 6-8, further comprising a switching logic device for switching the light source between two modes, wherein a first mode is the visible white light mode in which a laser diode 30 is off, the first LED is on, the second LED is on, and the third LED 36 is on, and wherein a second mode is an infrared excitation light and background light mode in which the laser diode is on, the first LED is on, the second LED is on, and the third LED is off.

10. The light source of any of claims 6-9, wherein the first LED is a green led, the second LED is a blue LED, and the third LED is a red LED.

## Patentansprüche

1. Verfahren zum Bereitstellen eines Farbabgleichs in einer Lichtquelle, wobei das Verfahren umfasst:
Bereitstellen einer ersten LED, einer zweiten LED und einer dritten LED, wobei die erste LED mit einem ersten DAC in Kommunikation steht, die zweite LED mit einem zweiten DAC in Kommunikation steht und die dritte LED mit einem dritten DAC in Kommunikation steht;
Bereitstellen einer Rechenvorrichtung, die mit dem ersten DAC, dem zweiten DAC und dem dritten DAC in Kommunikation steht, wobei die Rechenvorrichtung eine Speichervorrichtung aufweist, in der Zielintensitätswerte für die erste LED, die zweite LED und die dritte LED gespeichert sind;
Bereitstellen eines Farbsensors, der mit der Rechenvorrichtung in Kommunikation steht und dazu in der Lage ist, Licht im sichtbaren Lichtspektrum zu erfassen, und dazu in der Lage ist, der Rechenvorrichtung auf der Grundlage des erfassten Lichts Informationen bereitzustellen;
Abrufen von Farbwerten aus dem Farbsensor durch die Rechenvorrichtung;
Bestimmen durch die Rechenvorrichtung, ob die Farbintensitätswerte abgeglichen sind, um weißes Licht zu erzielen;
Anpassen des Stroms für den zweiten DAC und den dritten DAC, bis die Farbintensitätspegel abgeglichen sind, um weißes Licht bei einem Weißlichtintensitätspegel zu erzielen;
Einstellen eines neuen Rot-LED-Stromwerts, eines neuen Grün-LED-Stromwerts und eines neuen Blau-LED-Stromwerts durch Auslesen eines Zwischenintensitäts-DAC-Stromwerts für jede LED zwischen ihrem gegenwärtigen Intensitätspegel und ihrem Zielintensitätspegel aus der Speichervorrichtung, um den Weißlichtintensitätspegel in Richtung eines Zielintensitätspegels anzupassen, wobei die Zwischenintensitäts-DAC-Stromwerte zum Zeitpunkt der Kalibrierung in der Speichervorrichtung gespeichert und so eingestellt wurden, dass weißes Licht für den neuen Zwischenintensitätspegel erzielt wird; und
Wiederholen des Einstellschritts, bis der Zielintensitätspegel erzielt ist.

2. Verfahren nach Anspruch 1, wobei der Zielintensitätspegel von einem Benutzer ausgewählt wird.

3. Verfahren nach Anspruch 1 oder 2, wobei Rot-, Grün- und Blau-LED-Stromwerte für eine Mehrzahl von Intensitätspegeln in der Speichervorrichtung gespeichert sind.

4. Verfahren nach einem der Ansprüche 1-3, ferner umfassend das Umschalten der Lichtquelle zwischen zwei Modi, wobei ein erster Modus der Modus für sichtbares weißes Licht ist, in dem eine Laserdiode 30 ausgeschaltet ist, die erste LED eingeschaltet ist, die zweite LED eingeschaltet ist und die dritte LED 36 eingeschaltet ist, und wobei ein zweiter Modus ein Modus für Infrarot-Anregungslicht und Hintergrundlicht ist, in dem die Laserdiode eingeschaltet ist, die erste LED eingeschaltet ist, die zweite LED eingeschaltet ist und die dritte LED ausgeschaltet ist.

5. Verfahren nach einem der Ansprüche 1-4, wobei die erste LED eine grüne LED ist, die zweite LED eine blaue LED ist und die dritte LED eine rote LED ist.

6. Lichtquelle, umfassend:
eine erste LED (34), eine zweite LED (32) und eine dritte LED (36), wobei die erste LED mit einem ersten DAC (134) in Kommunikation steht, die zweite LED mit einem zweiten DAC (132) in Kommunikation steht und die dritte LED mit einem dritten DAC (136) in Kommunikation steht;
eine Rechenvorrichtung (62), die mit dem ersten DAC, dem zweiten DAC und dem dritten DAC in Kommunikation steht, wobei die Rechenvorrichtung eine Speichervorrichtung aufweist, in der Zielintensitätswerte für die erste LED, die zweite LED und die dritte LED gespeichert sind;
einen Farbsensor (60), der mit der Rechenvorrichtung in Kommunikation steht und dazu in der Lage ist, Licht im sichtbaren Lichtspektrum zu erfassen, und dazu in der Lage ist, der Rechenvorrichtung auf der Grundlage des erfassten Lichts Informationen bereitzustellen;
wobei die Rechenvorrichtung konfiguriert ist zum:
Abrufen von Farbwerten aus dem Farbsensor;
Bestimmen, ob die Farbintensitätswerte abgeglichen sind, um weißes Licht zu erzielen;
Anpassen des Stroms für den zweiten DAC und den dritten DAC, bis die Farbintensitätspegel abgeglichen sind, um weißes Licht bei einem Weißlichtintensitätspegel zu erzielen;
Einstellen eines neuen Rot-LED-Stromwerts, eines neuen Grün-LED-Stromwerts und eines neuen Blau-LED-Stromwerts durch Auslesen eines Zwischenintensitäts-DAC-Stromwerts für jede LED zwischen ihrem gegenwärtigen Intensitätspegel und ihrem Zielintensitätspegel aus der Speichervorrichtung, um den Weißlichtintensitätspegel in Richtung eines Zielintensitätspegels anzupassen, wobei die Zwischenintensitäts-DAC-Stromwerte zum Zeitpunkt der Kalibrierung in der Speichervorrichtung gespeichert und so eingestellt wurden, dass weißes Licht für den neuen Zwischenintensitätspegel erzielt wird; und
Wiederholen des Einstellschritts, bis der Zielintensitätspegel erzielt ist.

7. Lichtquelle nach Anspruch 6, wobei der Zielintensitätspegel durch einen Benutzer auswählbar ist.

8. Lichtquelle nach Anspruch 6 oder 7, wobei Rot-, Grün- und Blau-LED-Stromwerte für eine Mehrzahl von Intensitätspegeln in der Speichervorrichtung gespeichert sind.

9. Lichtquelle nach einem der Ansprüche 6-8, ferner umfassend eine Schaltlogikvorrichtung zum Umschalten der Lichtquelle zwischen zwei Modi, wobei ein erster Modus der Modus für sichtbares weißes Licht ist, in dem eine Laserdiode 30 ausgeschaltet ist, die erste LED eingeschaltet ist, die zweite LED eingeschaltet ist und die dritte LED 36 eingeschaltet ist, und wobei ein zweiter Modus ein Modus für Infrarot-Anregungslicht und Hintergrundlicht ist, in dem die Laserdiode eingeschaltet ist, die erste LED eingeschaltet ist, die zweite LED eingeschaltet ist und die dritte LED ausgeschaltet ist.

10. Lichtquelle nach einem der Ansprüche 6-9, wobei die erste LED eine grüne LED ist, die zweite LED eine blaue LED ist und die dritte LED eine rote LED ist.

## Revendications

1. Procédé pour appliquer un équilibre des couleurs à une source lumineuse, le procédé comprenant les étapes suivantes :
fournir une première LED [diode électroluminescente], une deuxième LED et une troisième LED, la première LED étant en communication avec un premier DAC [convertisseur numérique-analogique], la deuxième LED étant en communication avec un deuxième DAC et la troisième LED étant en communication avec un troisième DAC ;
fournir un dispositif informatique en communication avec le premier DAC, le deuxième DAC et le troisième DAC, le dispositif informatique ayant un dispositif de mémoire dans lequel sont stockées des valeurs d'intensité cibles pour la première LED, la deuxième LED et la troisième LED ;
fournir un capteur de couleur en communication avec le dispositif informatique, qui est en mesure de détecter la lumière dans le spectre de lumière visible et qui est en mesure de fournir des informations au dispositif informatique sur la base d'une lumière détectée ;
le dispositif informatique récupérant des valeurs de couleur à partir du capteur de couleur ;
le dispositif informatique déterminant si les valeurs d'intensité de couleur sont équilibrées pour obtenir une lumière blanche ;
régler le courant dans le deuxième DAC et dans le troisième DAC jusqu'à ce que les niveaux d'intensité de couleur soient équilibrées pour obtenir une lumière blanche à un niveau d'intensité de lumière blanche ;
définir une nouvelle valeur de courant de LED rouge, une nouvelle valeur de courant de LED verte et une nouvelle valeur de courant de LED bleue en lisant dans le dispositif de mémoire une valeur de courant de DAC d'intensité intermédiaire pour chaque LED entre son niveau d'intensité actuel et son niveau d'intensité cible pour régler le niveau d'intensité de lumière blanche sur un niveau d'intensité cible, dans lequel les valeurs de courant de DAC d'intensité intermédiaires étaient stockées au moment de l'étalonnage dans le dispositif de mémoire et définies pour obtenir une lumière blanche pour le nouveau niveau d'intensité intermédiaire ; et
répéter l'étape de définition jusqu'à ce que le niveau d'intensité cible soit atteint.

2. Procédé selon la revendication 1, dans lequel le niveau d'intensité cible est choisi par un utilisateur.

3. Procédé selon la revendication 1 ou 2, dans lequel des valeurs de courant de LED rouge, vert et bleu LED sont stockées dans le dispositif de mémoire pour une pluralité de niveaux d'intensité.

4. Procédé selon l'une des revendications 1 à 3, comprenant en outre la commutation de la source lumineuse entre deux modes, dans lequel un premier mode est le mode de lumière blanche visible où une diode laser 30 est éteinte, la première LED est allumée, la deuxième LED est allumée et la troisième LED 36 est allumée, et dans lequel un deuxième mode est un mode de lumière d'excitation infrarouge et de lumière d'arrière-plan où la diode laser est allumée, la première LED est allumée, la deuxième LED est allumée et la troisième LED est éteinte.

5. Procédé selon l'une des revendications 1 à 4, dans lequel la première LED est une LED verte, la deuxième LED est une LED bleue et la troisième LED est une LED rouge.

6. Source lumineuse comprenant :
une première LED (34), une deuxième LED (32) et une troisième LED (36), la première LED étant en communication avec un premier DAC (134), la deuxième LED étant en communication avec un deuxième DAC (132) et la troisième LED étant en communication avec un troisième DAC (136) ;
un dispositif informatique (62) en communication avec le premier DAC, le deuxième DAC et le troisième DAC, le dispositif informatique ayant un dispositif de mémoire dans lequel sont stockées des valeurs d'intensité cibles pour la première LED, la deuxième LED et la troisième LED ;
un capteur de couleur (60) en communication avec le dispositif informatique, qui est en mesure de détecter la lumière dans le spectre de lumière visible et qui est en mesure de fournir des informations au dispositif informatique sur la base d'une lumière détectée ;
le dispositif informatique étant configuré pour :
récupérer des valeurs de couleur à partir du capteur de couleur ;
déterminer si les valeurs d'intensité de couleur sont équilibrées pour obtenir une lumière blanche ;
régler le courant dans le deuxième DAC et dans le troisième DAC jusqu'à ce que les niveaux d'intensité de couleur soient équilibrées pour obtenir une lumière blanche à un niveau d'intensité de lumière blanche ;
définir une nouvelle valeur de courant de LED rouge, une nouvelle valeur de courant de LED verte et une nouvelle valeur de courant de LED bleue en lisant dans le dispositif de mémoire une valeur de courant de DAC d'intensité intermédiaire pour chaque LED entre son niveau d'intensité actuel et son niveau d'intensité cible pour régler le niveau d'intensité de lumière blanche sur un niveau d'intensité cible, dans laquelle les valeurs de courant de DAC d'intensité intermédiaires étaient stockées au moment de l'étalonnage dans le dispositif de mémoire et définies pour obtenir une lumière blanche pour le nouveau niveau d'intensité intermédiaire ; et
répéter l'étape de définition jusqu'à ce que le niveau d'intensité cible soit atteint.

7. Source lumineuse selon la revendication 6, dans laquelle le niveau d'intensité cible est sélectionnable par l'utilisateur.

8. Source lumineuse selon la revendication 6 ou 7, dans laquelle les valeurs de courant de LED rouge, verte et bleue sont stockées dans le dispositif de mémoire pour une pluralité de niveaux d'intensité.

9. Source lumineuse selon l'une des revendications 6 à 8, comprenant en outre une dispositif logique de commutation pour commuter la source lumineuse entre deux modes, dans laquelle un premier mode est le mode de lumière blanche visible où une diode laser 30 est éteinte, la première LED est allumée, la deuxième LED est allumée et la troisième LED 36 est allumée, et dans laquelle un deuxième mode est un mode de lumière d'excitation infrarouge et de lumière d'arrière-plan où la diode laser est allumée, la première LED est allumée, la deuxième LED est allumée et la troisième LED est éteinte.

10. Source lumineuse selon l'une des revendications 6 à 9, dans laquelle la première LED est une LED verte, la deuxième LED est une LED bleue et la troisième LED est une LED rouge.
